(19) Europäisches Patentamt
European Patent Office
Office européen des brevets

(11) Veröffentlichungsnummer: **0 267 873 B1**

# (12) EUROPÄISCHE PATENTSCHRIFT

(45) Veröffentlichungstag der Patentschrift: **17.02.93**

(51) Int. Cl.⁵: **C09B 48/00**, C08K 5/36, G03G 5/06

(21) Anmeldenummer: **87810634.3**

(22) Anmeldetag: **04.11.87**

## (54) Neue Thiochinacridone und Isothiochinacridone, Verfahren zu deren Herstellung und Verwendung.

(30) Priorität: **10.11.86 US 929299**

(43) Veröffentlichungstag der Anmeldung:
**18.05.88 Patentblatt 88/20**

(45) Bekanntmachung des Hinweises auf die Patenterteilung:
**17.02.93 Patentblatt 93/07**

(84) Benannte Vertragsstaaten:
**CH DE FR GB IT LI**

(56) Entgegenhaltungen:
**AU-B- 478 532**
**BE-A- 763 390**
**US-A- 3 474 020**

(73) Patentinhaber: **CIBA-GEIGY AG**
**Klybeckstrasse 141**
**CH-4002 Basel(CH)**

(72) Erfinder: **Rochat, Alain Claude, Dr.**
**Route de Schiffenen 38**
**CH-1700 Fribourg(CH)**
Erfinder: **Jaffe, Edward Ephraim, Dr.**
**3 Crenshaw Drive**
**Wilmington Delaware 19810(US)**
Erfinder: **Mizuguchi, Jin, Dr.**
**Route de Centre 17**
**CH-1723 Marly(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 267 873 B1

Rank Xerox (UK) Business Services
(3.10/3.5x/3.0.1)

## Beschreibung

Die Erfindung betrifft neue Thiochinacridone und Isothiochinacridone, ein Verfahren zu deren Herstellung sowie ihre Verwendung als photoleitfähige Substanzen und zum Färben von hochmolekularem organischem Material.

Chinacridone sind seit langem beschrieben und haben sich insbesondere als wertvolle Pigmente zum Einfärben von hochmolekularen organischen Materialien bewährt. Aus US-Patent 3 474 020 sind Chinacridone auch als photoleitfähige Verbindungen bekannt. Thiochinacridone dagegen sind bis anhin nicht beschrieben worden.

Gegenstand der vorliegenden Erfindung sind Verbindungen der Formeln I, II und III

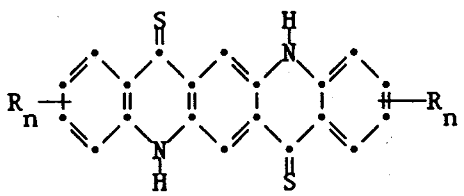
,

(I)

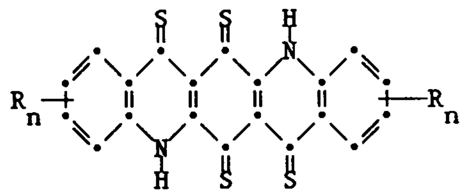
,

(II)

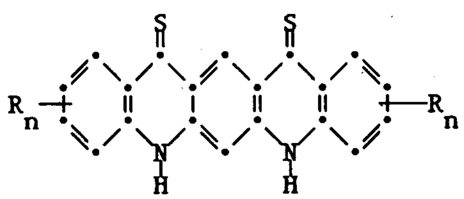
,

(III)

worin R -F, -Cl, -Br, $C_1$-$C_{18}$-Alkyl oder $C_1$-$C_3$-Alkoxy und n die Zahl 0, 1, 2 oder 3 bedeuten.

Bedeutet R $C_1$-$C_{18}$-Alkyl, so können die Alkylgruppen verzweigt oder unverzweigt sein und weisen vorzugsweise 1-12, insbesondere 1-6, und besonders bevorzugt 1-3 C-Atome, auf. Als Beispiele seien Methyl, Aethyl, Propyl, Isopropyl, Butyl, sec.-Butyl, tert.-Butyl, Pentyl, Isopentyl, tert.-Pentyl, Neopentyl, Hexyl, Neohexyl, Octyl, 2-Aethylhexyl, Decyl, Dodecyl und Stearyl genannt.

n stellt insbesondere die Zahl 0 oder 1 dar.

Von besonderem Interesse sind Verbindungen der Formeln I und II, worin R und n die oben angegebene Bedeutung haben.

Von ganz besonderem Interesse sind Verbindungen der Formel I, worin n die Zahl 0 oder 1 darstellt und R -F, -Cl, -Br, $-CH_3$ oder $-OCH_3$ bedeutet.

Zu den Verbindungen der Formeln I bis III gelangt man beispielsweise, indem man ein entsprechendes Chinacridon, Chinacridonchinon oder Isochinacridon der Formeln I bis III, worin die Schwefelatome durch Sauerstoff ersetzt sind, mit einem Schwefel abgebenden Mittel (Schwefelungsmittel) erwärmt. Es eignen sich die bekannten Schwefelungsmittel, wie Tetraphosphortrisulfid ($P_4S_3$), Tetraphosphorheptasulfid ($P_4S_7$), Tetraphosphordekasulfid ($P_4S_{10}$) sowie desssen Pyridinkomplex $P_4S_{10} \cdot 4C_5H_5N$, ferner Dithiaphosphaetane und insbesondere das 2,4-Bis-(4'-methoxyphenyl)-1,3-dithia-2,4-diphosphaetan-2,4-disulfid, das als Lawesson's Reagenz bekannt ist. Letzteres und Tetraphosphordekasulfid sind bevorzugt. Weitere Arylthionophosphinesulfide sind auch geeignet und beispielsweise im Uebersichtsreferat "Tetrahedron, Vol. 41, No. 22, S. 5061-5087, insbesondere S. 5062, (1985), M. P. Cava und M. I. Levinson" beschrieben. Die Umsetzung wird zweckmässig bei Temperaturen zwischen 50-250 °C mit einem Ueberschuss des Schwefelungsmittels, vorzugsweise zwischen 80 und 160 °C, und in einem inerten organischen Lösungsmittel, durchgeführt.

Als Lösungsmittel eignen sich beispielsweise aromatische Kohlenwasserstoffe, wie Benzol, Toluol, Xylol oder Tetrahydronaphthalin, chlorierte aromatische Kohlenwasserstoffe, wie Chlorbenzol und o-Dichlorbenzol, Aether, wie Aethylenglykoldimethyläther oder -diäthyläther, ferner Diäthylenglykoldimethyläther und -diäthyläther, Anisol, Dioxan oder Diphenyläther, oder auch Nitrile, wie Acetonitril oder Benzonitril, gegenüber Schwefelungsmittel inerte Amide, wie Hexamethylphosphorsäuretriamid, ferner Thioamide, wie z.B.

Dimethylthioformamid, Dimethylthioacetamid oder Tetraäthylsulfamid, oder Gemische der erwähnten Lösungsmittel.

Die erhaltenen Thiochinacridone bzw. Isothiochinacridone der Formeln I bis III können in der Regel durch Abfiltrieren isoliert werden. Je nach Verwendung ist eine Nachbehandlung erforderlich, um die chemische Reinheit zu erhöhen (z.B. wie Rekristallisation oder Sublimation) und/oder um die Kristallform zu verändern (z.B. wie Konditionierung in einem organischen Lösungsmittel). Dazu erweisen sich beispielsweise folgende Lösungsmittel als geeignet: Durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Aceton, Methyläthylketon oder Cyclohexanon, Aether, wie Aethylenglykolmonomethyl- oder -monoäthyläther oder Tetrahydrofuran, Amide, wie Dimethylformamid, Dimethylacetamid oder N-Methyl-pyrrolidon, ferner Alkohole, wie Methanol oder Aethanol, Dimethylsulfoxyd, Sulfolan, Acetonitril, Benzonitril und Methyl- sowie Aethylacetat. Nach dieser Lösungsmittelbehandlung liegen die Verbindungen der Formeln I bis III manchmal schon in der gewünschten Kristallform vor, so dass sich eine weitere thermische Nachbehandlung erübrigt, beispielsweise wie auf Seite 8 angegeben.

Je nach Art der Verbindungen der Formeln I bis III können diese Verbindungen als photoleitfähige Substanzen für elektrophotographische Photorezeptoren oder Sonnenbatterien und als Pigmente verwendet werden.

Von besonderem Interesse sind die Verbindungen der Formeln I bis III, insbesondere der Formel I, als photoleitfähige Substanzen in elektrophotographischen Photorezeptoren. Solche Photorezeptoren bestehen aus einer leitfähigen Unterlage und einem Photoleiter, der im Dunkeln isolierend ist, aber unter Belichtung leitend wird. Der Photoleiter kann aus einer oder mehreren Schichten bestehen. Im Fall einer einzigen Schicht ist mindestens eine photoleitfähige Substanz in mindestens einem Bindemittel dispergiert oder unmittelbar auf eine leitende Unterlage aufgedampft. Ein mehrschichtiger Photoleiter besteht bevorzugt aus mindestens einer photoleitfähigen Schicht, enthaltend eine oder mehrere photoleitfähige Substanzen, und mindestens einer ladungstransportierenden Schicht.

Ein weiterer Gegenstand der vorliegenden Erfindung ist demnach ein elektrophotographischer Photorezeptor, bestehend aus mindestens einer leitfähigen Unterlage, einer photoleitfähigen Schicht und einer ladungstransportierenden Schicht, wobei in mindestens einer dieser Schichten mindestens eine Verbindung der Formeln I bis III enthalten ist.

Die leitfähige Unterlage kann aus einer Metallplatte oder -folie bestehen, die roh oder z.B. durch Aufrauhen vorbehandelt ist und z.B. aus Aluminium, Zink, Magnesium, Kupfer oder einer Legierung dieser Metalle besteht. Im Falle des Aluminiums kann die Vorbehandlung in einem Anodisieren bestehen. Als Unterlagen kommen auch aluminiumbedampfte Kunststoff-Folien sowie Polymerfilme mit metallisierter Oberfläche in Frage.

Der Photoleiter enthält als photoleitfähige Verbindungen mindestens eine Verbindung der Formeln I bis III und als ladungstransportierende Substanzen Verbindungen, wie z.B. Hydrazone oder Pyrazoline, welche im Polymer-Bindemittel gelöst sind. Ein solcher Aufbau erlaubt nach vorgängiger statischer Aufladung und bildmässiger Belichtung die Erzeugung eines entsprechenden Musters aufgeladener und entladener Stellen (latentes Bild), welches nach bekannten Verfahren der Reprographie in ein sichtbares Abbild übergeführt werden kann.

Die Belichtung kann mit Licht im sichtbaren Wellenbereich erfolgen. Es ist aber ein besonderer Vorzug der Verbindungen der Formeln I bis III, dass sie auch Strahlung im nahen Infrarot-Bereich zu absorbieren vermögen und dass sie in diesem Wellenlängenbereich auch Photoleitung zeigen. Von besonderem Interesse ist dabei der Bereich 700-900 nm, in welchem die Galliumarsenid-Laser arbeiten.

Zur Erhaltung des statischen Potentials an Stellen, die nicht belichtet werden, tragen die Verbindungen der Formeln I bis III dadurch bei, dass sie einen hohen Dunkelwiderstand aufweisen.

Besteht der Photoleiter aus einer einzigen Schicht, so enthält diese eine oder mehrere Verbindungen der Formeln I bis III zweckmässig in fein verteilter Form, gegebenenfalls zusammen mit ladungstransportierenden Substanzen, in einem organischen Bindemittel. Das Bindemittel ist zweckmässig filmbildend, isolierend und adhäsiv. Je nach Anwendung ist es löslich in organischen Lösungsmitteln oder in basischen Mischungen organischer Lösungsmittel, die gegebenenfalls Wasser enthalten. Besonders geeignet sind Bindemittel auf der Basis von Polykondensations- und Polyadditionsprodukten, wie Polyamiden, Polyurethanen, Polyestern, Epoxyharzen, Phenoxyharzen, Polyketonen, Polycarbonaten, Polyvinylketonen, Polystyrolen, Polyvinylcarbazolen, Polyacrylamiden, Polymethylmethacrylaten, Polyvinylbutyraten, Polyvinylchloriden sowie Copolymerisaten, wie z.B. Styrol-Maleinsäureanhydrid-Copolymeren oder Styrol-Methacrylsäure-Methacrylsäureester-Copolymeren.

Besteht der Photoleiter aus mehreren Schichten, so sind Doppelschichten von besonderem Interesse. Für diesen Fall wird auf die leitfähige Unterlage zuerst eine photoleitfähige und auf diese eine zweite

ladungstransportierende Schicht aufgebracht. Das Aufbringen der Schichten kann auch in umgekehrter Reihenfolge durchgeführt werden. Eine der Schichten, vorzugsweise die ladungserzeugende Schicht, enthält mindestens eine Verbindung der Formeln I bis III. Diese kann in einem organischen Bindemittel gelöst oder fein verteilt sein. Die Applikation auf die leitfähige Unterlage erfolgt beispielsweise durch Auftragen einer Lösung beziehungsweise Dispersion der Bindemittel-Farbkörper-Mischung in einem organischen Lösungsmittel und anschliessendes Verdampfen des Lösungsmittels. Man kann die Verbindung der Formeln I bis III aber auch auf die leitfähige Unterlage aufdampfen.

Die zweite Schicht enthält eine oder mehrere ladungstransportierende Substanzen, vorzugsweise gelöst oder dispergiert in einem organischen Bindemittel. Als ladungstransportierende Substanzen kommen die verschiedensten aromatischen, vorzugsweise stickstoffhaltigen Verbindungen in Frage, wie Hydrazone oder aromatische Amine, die gegebenenfalls Alkylidenbrücken oder -reste enthalten. Beispielsweise handelt es sich um die in der Deutschen Offenlegungsschrift 34 47 685 auf den Seiten 57-65 beschriebenen Substanzen.

Obwohl die erfindungsgemässen Verbindungen der Formeln I bis III die minimale Absorption von 750 nm, die für die Laser-Aufzeichnung benötigt wird, nicht aufweisen, sind sie als photoleitende Substanzen für einen derartigen Einsatz verwendbar. Hierfür wurde eine Methode gefunden, die es erlaubt, diese Absorption in für die Elektrophotographie entscheidendem Masse nach längeren Wellenlängen zu verschieben. Diese ist überraschend, da die gleichartige Lösungsmittelbehandlung von Chinacridonen im allgemeinen zu einer Verschiebung nach kurzwelligeren Wellenlängen führt. Die genannte Verschiebung geschieht zweckmässig dadurch, dass man den wie oben beschrieben erhaltenen photographischen Photorezeptor während 1-2 Stunden einem Lösungsmitteldampf aussetzt, wie z.B. Aceton-, Tetrahydrofuran-, Dimethylformamid-, Methanol-, Acetonitril-, 1-Acetoxy-2-äthoxyäthan-,Dimethylsulfoxid- oder Aethylacetatdampf.

Aus der Publikation von K. Arishima et al., Appl. Phys. Letters 40 (3), S. 279 (1982), ist diese Methode zur Verschiebung der Wellenlänge für bestimmte Phthalocyanine bekannt. Man erreicht dabei eine Verscheibung nach längeren Wellenlängen um ca. 90 nm. Die Anwendung dieser Methode auf Materialien, welche die erfindungsgemässen Verbindungen der Formeln I bis III enthalten, ergibt eine Verschiebung nach längeren Wellenlängen um ca. 70 und mehr nm. Diese Lösungsmittelbehandlung bewirkt eine Erhöhung der Photoleitfähigkeit um ca. 3 bis 100 Einheiten.

Dieselbe spektrale Verschiebung, sowie ähnliche Werte für den Dunkel-Widerstand und den Photostrom werden auch mit den gepulverten Verbindungen der Formeln I bis III erhalten; sie werden zuerst gemahlen, dann mit einem Lösungsmittel, wie z.B. Aethylacetat, behandelt und anschliessend mit Hilfe eines Bindemittels auf eine Unterlage aufgetragen.

Ein weiterer Gegenstand der Erfindung betrifft demnach die Herstellung eines elektrophotographischen Photorezeptors, dadurch gekennzeichnet, dass man eine Verbindung der Formeln I bis III mit einem organischen Bindemittel auf eine leitende Unterlage aufträgt oder im Vakuum aufdampft, die so hergestellte Schicht mit einem organischen Lösungsmittel in flüssiger oder gasförmiger Form behandelt und anschliessend eine zweite Schicht, enthaltend eine aromatische, stickstoffhaltige Verbindung, aufbaut. Als (ladungstransportierende) aromatische stickstoffhaltige Verbindungen kommen z.B. solche der vorerwähnten Art in Betracht.

Zur Verbesserung der physikalischen Eigenschaften der Schichten können sowohl die photoleitfähige als auch die ladungstransportierende Schicht noch Zusätze, wie Egalisiermittel, oberflächenaktive Mittel oder Weichmacher, enthalten.

Die erfindungsgemässen Verbindungen der Formeln I, II und III, insbesonders aber der Formel I, können ferner auch als Pigmente zum Färben von hochmolekularem organischem Material verwendet werden.

Je nach Verwendungszweck kann man die erfindungsgemässen Verbindungen in eine deckendere oder transparentere Pigmentform überführen.

Wird eine deckendere Pigmentform gewünscht, so erweist sich in der Regel eine thermische Nachbehandlung in Wasser oder in einem organischen Lösungsmittel als zweckmässig, gegebenenfalls unter Druck. Vorzugsweise verwendet man organische Lösungsmittel, wie durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picoline oder Chinoline, ferner Ketone, wie Cyclohexanon, Alkohole, wie Isopropanol, Butanole oder Pentanole, Aether, wie Aethylenglykolmonomethyl- oder -monoäthyläther, Amide, wie Dimethylformamid oder N-Methylpyrrolidon, sowie Dimethylsulfoxid oder Sulfolan. Man kann die Nachbehandlung auch in Wasser, gegebenenfalls unter Druck, in Gegenwart von organischen Lösungsmitteln und/oder mit Zusatz von oberflächenaktiven Substanzen durchführen.

Die Wahl des Lösungsmittels und der Temperatur für eine derartige thermische Nachbehandlung hängt stark von der Löslichkeit der zu behandelnden Verbindung in diesem Lösungsmittel ab, so dass es für jede

erfindungsgemäss in Frage kommende Verbindung zweckmässig ist, die optimalen Nachbehandlungsbedingungen, wie Lösungsmittelauswahl, Konzentration und Temperatur, im voraus auszuwählen.

Hochmolekulare organische Materialien, die mit den erfindungsgemässen Verbindungen gefärbt bzw. pigmentiert werden können, sind z.B. Celluloseäther und -ester, wie Aethylcellulose, Nitrocellulose, Celluloseacetat oder Cellulosebutyrat, natürliche Harze oder Kunstharze, wie Polymerisationsharze oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Verbindungen als Toner oder in Form von Präparaten einzusetzen. Bezogen auf das zu pigmentierende hochmolekulare organische Material kann man die erfindungsgemässen Verbindungen beispielsweise in einer Menge von 0,1 bis 30 Gew.% einsetzen.

Die Pigmentierung der hochmolekularen organischen Substanzen mit den erfindungsgemässen Verbindungen erfolgt beispielsweise derart, dass man eine solche Verbindung gegebenenfalls in Form von Masterbatches diesen Substraten unter Verwendung von Walzwerken, Misch- oder Mahlapparaten zumischt. Das pigmentierte Material wird hierauf nach an sich bekannten Verfahren, wie Kalandrieren, Pressen, Strangpressen, Streichen, Giessen oder Spritzguss, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung der erfindungsgemässen Verbindung in die Polymeren eingearbeitet werden. Es ist ferner möglich, zwecks Erzielung verschiedener Farbtöne den hochmolekularen, organischen Stoffen neben den erfindungsgemässen Verbindungen noch Füllstoffe bzw. andere farbgebende Bestandteile, wie Weiss-, Bunt- oder Schwarzpigmente, in beliebigen Mengen zuzufügen.

Zum Pigmentieren von Lacken, Anstrichstoffen und Druckfarben werden die hochmolekularen organischen Materialien und die erfindungsgemässen Verbindungen gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Die erhaltenen Färbungen, beispielsweise in Kunststoffen, Fasern, Lacken oder Drucken, zeichnen sich im allgemeinen durch mittelmässige bis gute allgemeine Eigenschaften, wie Farbstärke, Dispergierbarkeit, Ueberlackier-, Migrations-, Hitze-, Licht- und Wetterbeständigkeit, sowie Glanz aus.

Die nachstehenden Beispiele erläutern die Erfindung:

Beispiel 1: Synthese von Chino[2,3-b]acridin-7,14-dithion (= Dithiochinacridon)

7,95 g rohes, unsubstituiertes Chinacridon werden in 175 ml o-Dichlorbenzol suspendiert und mit 12,4 g Lawesson's Reagenz (= 2,4-Bis-(4'-methoxyphenyl)-1,3-dithia-2,4-diphosphaetan-2,4-disulfid) versetzt. Die erhaltene Suspension wird unter Stickstoff-Atmosphäre auf 143 °C erwärmt und während 3 Stunden bei dieser Temperatur gehalten. Man kühlt das Reaktionsgemisch auf 100 °C, filtriert die erhaltene grün-blaue Verbindung und wäscht sie nacheinander mit warmem o-Dichlorbenzol, kaltem Methanol und Aceton. Nach der Trocknung bei 100 °C im Vakuumschrank erhält man 8,93 g eines Rohproduktes, das durch Umkristallisation in Dimethylsulfoxid (= DMSO) wie folgt gereinigt wird:
Unter Spülung mit Argon suspendiert man 5,0 g des trockenen Rohproduktes in 150 ml reinem DMSO und heizt das Gemisch auf 140 °C. Nachdem das Produkt in Lösung gegangen ist, kühlt man diese Lösung langsam bis auf ca. 80 °C, wobei das Produkt zu kristallisieren beginnt. Nach etwa 1 Stunde bei 80 °C wird die Suspension auf ca. 70 °C gekühlt und während 15 Stunden bei dieser Temperatur gehalten. Anschliessend filtriert man das auskristallisierte Produkt bei 70 °C und wäscht es sehr sorgfältig mit warmem Methanol, dann mit kaltem Aceton. Nach Trocknung bei 100 °C erhält man 3,90 g eines Pulvers, entsprechend einer Ausbeute von 80,9 % der Theorie (bezogen auf das eingesetzte Chinacridon), das einen Schmelzpunkt von über 300 °C aufweist und der folgenden Struktur

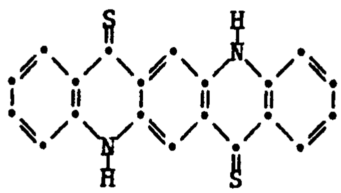

entspricht.

| Elementaranalyse: | Berechnet | Gefunden |
|---|---|---|
| | C 69,74 % | C 69,14 % |
| | H 3,51 % | H 3,62 % |
| | N 8,13 % | N 7,92 % |
| | S 18,62 % | S 18,34 % |

Beispiel 2:

3,12 g (0,01 Mol) γ-Chinacridon werden in 50 ml Hexamethylphosphorsäuretriamid suspendiert und mit 4,85 g Lawesson's Reagenz versetzt. Die erhaltene Suspension wird innert 30 Minuten auf 130-140 °C erwärmt und bei dieser Temperatur während 265 Minuten weiter gerührt. Die auf 80 °C abgekühlte Suspension wird dann in 300 ml Wasser gegossen, das erhaltene Gemisch wird während 30 Minuten gerührt, und der erhaltene Rückstand wird abfiltriert, mit Wasser und dann mit Methanol gewaschen und bei 80 °C getrocknet. Man erhält 3,35 g (97,4 % der Theorie) eines tief grünen Produktes vom Schmelzpunkt über 300 °C, enthaltend gemäss Elementaranalyse 18,5 % Schwefel (berechnete Menge 18,6 %).

Zur Analyse wird der nach Ausgiessen in Wasser oben erhaltene Rückstand bei Raumtemperatur in kaltem Dimethylformamid suspendiert, die erhaltene Lösung wird klarfiltriert, und das erhaltene Filtrat wird mit Methanol verdünnt. Das so ausgefallene Produkt wird abfiltriert und bei 80 °C getrocknet. Das Produkt entspricht der im Beispiel 1 angegebenen Formel.

| Elementaranalyse: | Berechnet | Gefunden |
|---|---|---|
| | C 69,74 % | 68,50 % |
| | H 3,51 % | 3.50 % |
| | N 8,13 % | 8,20 % |
| | S 18,62 % | 18,20 % |

Extinktionskoeffizient bei 3 Wellenlängen des sichtbaren Bereichs (ε-Wert bei $\lambda_{max}$) in Dimethylformamid:

ε-Wert: 13075 bei λ 647 nm,
10172 bei λ 594 nm,
4344 bei λ 552 nm.

Röntgenbeugungsdiagramme (20° Winkel):
2 starke Linien bei 8,8 und 24,9;
4 mittlere Linien bei 12,7, 12,8, 17,7 und 27,1;
4 schwache Linien bei 18,5, 19,5, 21,6 und 24,2.

Beispiel 3:

1,56 g (0,005 Mol) Chinacridon werden in 25 ml Dimethylthioformamid suspendiert, mit 2,43 g (0,006 Mol) Lawesson's Reagenz versetzt, innerhalb 45 Minuten auf 145°C erwärmt und während 4 ½ Stunden bei dieser Temperatur gerührt. Die auf 60°C abgekühlte Mischung wird dann in 150 ml Methanol gegossen, und die resultierende Suspension wird bei Raumtemperatur gerührt. Der Festanteil wird abfiltriert, mit Methanol bis farblos gewaschen und bei 80°C getrocknet. Man erhält 1,65 g (95,9 % der Theorie) eines Produktes, das der im Beispiel 1 angegebenen Formel entspricht und gemäss Elementaranalyse 17,5 % Schwefel (berechnet: 18,6 %) enthält.

Beispiel 4:

1,56 g (0,005 Mol) Chinacridon werden in 50 ml Xylol und 5 ml Hexamethylphosphorsäuretriamid suspendiert, mit 2,43 g (0,006 Mol) Lawesson's Reagenz versetzt, das erhaltene Gemisch wird auf 130 - 140°C erwärmt und bei dieser Temperatur gerührt. Die auf 50°C abgekühlte Reaktionsmischung wird zur Abtrennung kleiner Mengen unreagierten Materials filtriert. Das Filtrat wird in 200 ml Methanol gegossen, und das ausgefällte Produkt wird abfiltriert, mit Methanol gewaschen und getrocknet. Man erhält 1,42 g (82,6 % der Theorie) eines tief grünen Produktes, das im wesentlichen das gleiche IR-Spektrum wie dasjenige der gemäss Beispiel 2 erhaltenen Verbindung aufweist, jedoch ein anderes Röntgenbeugungsdiagramm besitzt:
Drei starke Linien bei 8,4 12,8 und 26,0;
zwei starke Linien bei 12,2 und 27,5;
acht schwache Linien bei 7,3, 10,2, 14,5, 16,7, 18,7, 22,2 22,7 und 24,8.

Dieses Produkt stellt daher eine andere Kristallmodifikation des Dithiochinacridons dar.

Beispiel 5:

3,18 g (0,01 Mol) 2,9-Dichlorchinacridon werden in 100 ml Hexamethylphosphorsäuretriamid suspendiert, das Gemisch wird mit 4,85 g (0,012 Mol) Lawesson's Reagenz versetzt und innerhalb 20 Minuten auf 140 - 145°C erwärmt. Die Mischung wird während vier Stunden bei dieser Temperatur gerührt, dann auf 60°C abgekühlt und in 500 ml Wasser gegossen. Die entstandene Suspension wird während 30 Minuten bei Raumtemperatur gerührt, das Produkt wird abfiltriert, mit Wasser, dann mit Methanol gewaschen. Man erhält 4,5 g eines rohen Produktes, das aus 375 ml Dimethylformamid rekristallisiert wird. Dabei werden 2,53 g reines Produkt erhalten, welches der folgenden Formel entspricht:

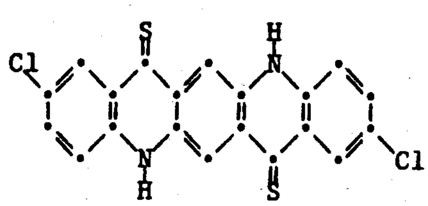

.

Elementaranalyse für Schwefel in %:

| | |
|---|---|
| Berechnet: | 15,5 % |
| Gefunden: | 14,3 %. |

Beispiel 6:

3,81 g (0,01 Mol) 4,11-Dichlorchinacridon werden in 100 ml o-Dichlorbenzol suspendiert und mit 4,85 g (0,012 Mol) Lawesson's Reagenz unter $N_2$-Atmosphäre versetzt; das entstandene Reaktionsgemisch wird auf 145°C erwärmt und während 4 Stunden bei dieser Temperatur gerührt. Die auf Raumtemperatur abgekühlte Suspension wird in 200 ml Methanol gegossen, und das erhaltene Produkt wird abfiltriert, mit Methanol bis farblos gewaschen und bei 80°C getrocknet. Man erhält 4,12 g (100 % der Theorie) eines Produktes, das gemäss Elementaranalyse 15,4 % Schwefel (berechnet: 15,5 %) enthält und folgende Formel aufweist

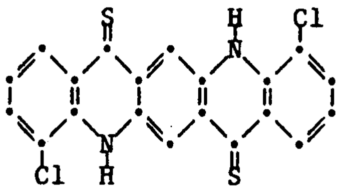

.

Beispiel 7:

3,4 g (0,01 Mol) 2,9-Dimethylchinacridon werden in 50 ml Hexamethylphosphorsäuretriamid suspendiert und mit 4,85 g (0,012 Mol) Lawesson's Reagenz versetzt, die entstandene Mischung wird innerhalb 12 Minuten auf 135 °C erwärmt und bei dieser Temperatur während 5 Stunden gerührt. Die auf 60 °C abgekühlte Mischung wird in 200 ml Methanol gegossen, und die erhaltene Suspension wird während 15 Minuten bei Raumtemperatur gerührt, das Produkt wird abfiltriert, mit Methanol gewaschen und bei 80 °C getrocknet. Man erhält 3,54 g (95,2 % der Theorie) eines tief grünen Produktes, das gemäss Elementaranalyse 16,5 % Schwefel (berechnet: 17,2 %) enthält und die folgende Formel aufweist

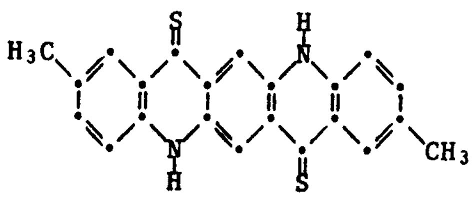

.

Beispiel 8:

1,86 g (0,005 Mol) 2,9-Dimethoxychinacridon werden in 100 ml o-Dichlorbenzol suspendiert, das entstandene Gemisch wird mit 2,43 g (0,006 Mol) Lawesson's Reagenz versetzt, unter Stickstoff auf 138 - 142 °C erwärmt und während 4 Stunden bei dieser Temperatur gerührt.

Die erhaltene Reaktionsmischung wird auf Raumtemperatur abgekühlt, in 200 ml Methanol gegossen, und das erhaltene Produkt wird abfiltriert, mit Methanol bis farblos gewaschen und bei 80 °C getrocknet. Man erhält 2,02 g (100 % der Theorie) eines tief grünen Produktes, das nach Rekristallisation in Dimethylacetamid gemäss Elementaranalyse 15,3 % Schwefel (berechnet: 15,8) enthält und der folgenden Struktur entspricht

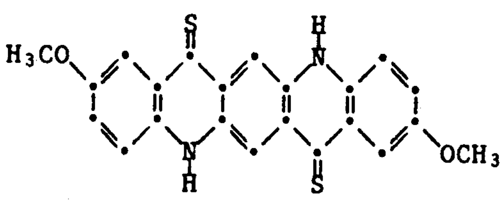

.

Beispiel 9:

1,56 g (0,005 Mol) Isochinacridon werden in 50 ml Hexamethylphosphorsäuretriamid suspendiert, mit 2,43 g (0,006 Mol) Lawesson's Reagenz versetzt, das Gemisch wird auf 140 °C erwärmt und bei dieser Temperatur während 4 Stunden gerührt. Die auf Raumtemperatur abgekühlte Reaktionsmischung wird in 200 ml Methanol gegossen und während 30 Minuten gerührt. Das erhaltene Produkt wird filtriert, mit Methanol gewaschen und getrocknet. Man erhält 1,81 g eines Rohproduktes.

Zur Reinigung werden 0,5 g Rohprodukt in 100 ml Dimethylacetamid bei 50 - 60 ° C gelöst, die erhaltene Lösung wird klarfiltriert, und das Filtrat wird mit 900 ml Methanol versetzt. Das ausgefallene Produkt wird abfiltriert und bei 80 ° C getrocknet. Man erhält 0,35 g eines tief roten Produktes, das gemäss Elementaranalyse 18,8 % Schwefel (berechnet: 18,6 %) enthält, die folgende Formel

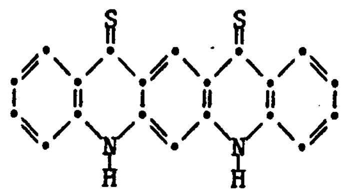

und molare Extinktionskoeffizienten ($\epsilon$-Werte bei $\lambda_{max}$) von 10021 (bei $\lambda$ 563 nm) und 9914 (bei $\lambda$ 541 nm) in Dimethylformamidlösung aufweist.

Beispiel 10 (Thionierung von Chinacridonchinon):

6,85 g gereinigtes Chinacridonchinon (durch Behandlung von rohem Chinacridonchinon in Aceton am Rückfluss erhalten) werden in einem Gemisch von 140 ml o-Dichlorbenzol und 14 ml Hexamethylphosphorsäuretriamid suspendiert, und das entstandene Gemisch wird mit 19,8 g Lawesson's Reagenz versetzt. Die so erhaltene Suspension wird auf 140 ° C unter Stickstoff-Atmosphäre erwärmt und während 12 Std. bei dieser Temperatur gehalten. Man kühlt dann das Reaktionsgemisch auf 60 ° C ab, filtriert das oliv-grüne Produkt und wäscht es nacheinander mit warmem o-Dichlorbenzol, kaltem Dimethylformamid und Wasser. Nach Trocknung bei 90 ° C im Vakuumschrank werden 6,72 g (82,7 % der Theorie) eines festen Rohproduktes erhalten, das gemäss Verbrennungsanalyse 26,9 % Schwefel (Theorie: 31,54 %) enthält und wie folgt gereinigt wird: 4,0 g Rohprodukt werden in 100 ml N-Methylpyrrolidon (NMP) unter Stickstoff auf 120 ° C erwärmt und während 6 Std. bei dieser Temperatur gehalten. Das erhaltene unlösliche Produkt wird bei dieser Temperatur abfiltriert und nacheinander mit kaltem NMP, kaltem Methanol und Wasser gewaschen. Nach Trocknung bei 90 ° C im Vakuum erhält man 2,29 g eines gereinigten Produktes, das einen Schmelzpunkt von über 300 ° C und ein Molekulargewicht von 406 (massenspektrometrische Bestimmung) mit der folgenden Struktur

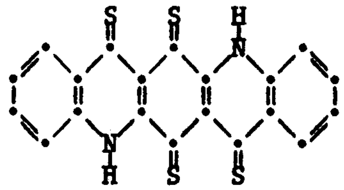

aufweist.

Beispiel 11:

In einem 4-Hals-Rundkolben (mit Rührer, Thermometer und Rückfluss-Kühler) werden 1,74 g (0,005 mol) 2,9-Difluorchinacridon, 2,43 g (0,006 mol) Lawesson's Reagenz und 50 ml Hexamethylphosphoramid suspendiert. Die erhaltene Suspension wird innert 15 Minuten auf 140-145 ° C erwärmt. Bei etwa 85 ° C wird das gelblich rote Reaktionsgemisch braun-rot, bei 100 ° C kirschrot, bei 125 ° C blau-violett, bei 130 ° C dunkel blau und bei 145 ° C dunkel grün. Die Suspension wird während 4,25 Stunden bei 140-145 ° C gehalten, dann auf 60 ° C gekühlt und unter Rühren in 200 ml Methanol gegossen. Das erhaltene Gemisch wird während 20 Minuten gerührt, und der erhaltene Rückstand wird abfiltriert, mit Methanol gut gewaschen (bis das Filtrat farblos ist) und dann bei 80 ° C getrocknet. Man erhält 1,76 g (92,6 % der Theorie) 2,9-Difluorodithiochinacridon als dunkel grünes Produkt, enthaltend gemäss Elementaranalyse 15,4 % Schwefel (berechnete Menge 16,8 %).

Beispiel 12:

In der gleichen Apparatur wie in Beispiel 11 werden 3,48 g (0,01 mol) 4,11-Difluorchinacridon, 4,86 g (0,012 mol) Lawesson's Reagenz und 100 ml o-Dichlorbenzol suspendiert. Das Gemisch wird innert 30 Minuten unter Rühren auf 140-145° C erwärmt, wobei das orange-braune Gemisch, insbesonders über 100° C, braun wird. Die Temperatur wird während 5 Stunden auf 140-145° C gehalten, wobei das ausgefallene dunkle Produkt schwarz wird. Das Gemisch wird auf 60° C gekühlt und in 200 ml Methanol unter Rühren gegossen. Das erhaltene Gemisch wird dann während einer Stunde gerührt, und der enthaltene Rückstand wird filtriert, mit Methanol gewaschen (bis das Filtrat farblos ist) und bei 80° C getrocknet. Man erhält 3,74 g (98,4 % der Theorie) 4,11-Difluorodithiochinacridon als grauschwarzes Produkt, enthaltend gemäss Elementaranalyse 15,8 % Schwefel (berechnete Menge: 16,8 %).

Beispiel 13:

0,5 g des gemäss Beispiel 1 erhaltenen Pigments werden in 15 g einer 7,5 gew.-%igen Lösung von Lucite® 41 (einem Polymethylmethacrylat, hergestellt von DuPont) in Methyläthylketon mit 70 g Glaskugeln von 4-5 mm Durchmesser in einer Flasche von 50 ml Inhalt während 16 Std. auf einer Vibrationskugelmühle (Typ Vibratom® der Firma Siebtechnik in Mühlheim/Ruhr) dispergiert. Nach Abtrennen der Glaskugeln wird die Pigmentdispersion mit einem Ziehstab von nominell 150 µm Nassfilmdicke auf eine Aluminiumunterlage aufgestrichen. Nach der Trocknung resultiert eine Schicht mit einer Dicke von ca. 15 µm, welche elektrophotographische Eigenschaften zeigt (E 1/2 ca. 15 $\mu J/cm^2$).

Beispiel 14:

0,3 g des Pigments vom Beispiel 1 werden in einem Gemisch von 10 g Aethanol und Methyläthylketon (2:1 in Volumen), enthaltend 0,2 g Aethylcellulose, aufgenommen. Die Suspension wird dann während 5 Std. mit Glaskugeln gemahlen und anschliessend mit einem Ziehstab auf eine Aluminiumplatte aufgetragen (= photoleitfähige resp.ladungserzeugende Schicht). Diese Schicht wird bei 50° C während 3 Std. getrocknet. Die Schichtdicke beträgt 6 µm. Eine zweite Schicht, bestehend aus einem Gemisch von 0,6 g eines Hydrazons der Formel

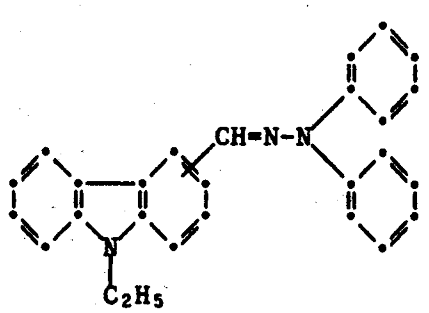

und 0,9 g Lucite® 41 in 11 g Methyläthylketon, wird aufgetragen und bei 50° C während 15 Std. getrocknet. Die Schichtdicke beträgt 10 bis 15 µm. Dieser Photorezeptor zeigt eine Empfindlichkeit (E 1/2) von 6 $\mu J/cm^2$ und die Aufladbarkeit beträgt 430 Volt.

Beispiel 15:

Das Pigment vom Beispiel 1 wird mit einer Geschwindigkeit von 5 Å/Sek unter einem Vakuum von $1{,}3 \cdot 10^{-6}$ bar auf eine Aluminium-Unterlage aufgedampft. Die erhaltene Schichtdicke beträgt 2000 bis 3000 Å. Dieser Film wird bei Raumtemperatur während 1 Std. dem Dampf von Aceton ausgesetzt. Anschliessend wird eine zweite Schicht von derselben Zusammensetzung wie im Beispiel 14 aufgetragen. Der Film zeigt eine Absorption bei 770 nm, was eine Verschiebung der Absorption um 70 nm ergibt (Absorption des entsprechenden Filmes, der durch Acetondampf nicht behandelt wurde: 700 nm).

Beispiel 16:

Das Pigment vom Beispiel 1 wird wie im Beispiel 15 aufgedampft und der erhaltene Film während 1 Std. dem Dampf von Dimethylformamid ausgesetzt. Eine zweite Schicht wird darüber aufgetragen, deren Zusammensetzung derjenigen von Beispiel 14 entspricht. Der Film zeigt eine Absorption bei 770 nm.

Beispiel 17:

0,4 g des Pigmentes vom Beispiel 1 werden in 10 ml destilliertem Wasser mit 40 g Glaskugeln von 1 mm Durchmesser während 2 Tagen gemahlen. Das erhaltene Produkt wird filtriert und bei 50 ° C während 24 Std. getrocknet, anschliessend während 1 Std. in Aethylacetat suspendiert, und wieder filtriert und getrocknet. Die Herstellung der Monoschicht erfolgt wie im Beispiel 13. Der Film zeigt eine Absorption bei 770 nm.

Beispiel 18:

Mit dem Pigment vom Beispiel 1, welches wie im Beispiel 17 vorbehandelt wird, erzeugt man eine Doppel-Schicht nach der Vorschrift von Beispiel 14. Die so aufgebaute Schicht zeigt günstige elektrophotographische Eigenschaften.

Beispiel 19:

0,5 g des Pigmentes gemäss Beispiel 10 werden in einem AM-Lack [10 g, Gemisch aus einem Alkyd- und einem Melaminharz in einem Lösungsmittelgemisch Xylol und Methylglykol] mit Glaskugeln während 15 Stunden wie im Beispiel 13 dispergiert. Nach Abtrennen der Glaskugeln wird die Pigmentdispersion mit einem Ziehstab auf eine Aluminiumunterlage wie im Beispiel 13 aufgestrichen (photoleitfähige Schicht). Eine zweite Schicht (ladungstransportierende Schicht) wird dann gemäss Beispiel 14 aufgetragen. Dieser Photorezeptor zeigt günstige elektrophotographische Eigenschaften.

## Patentansprüche

**1.** Verbindungen der Formeln I, II und III

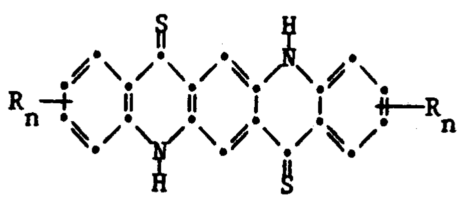 ,

(I)

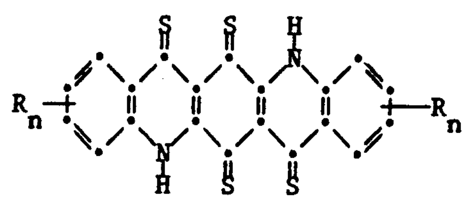 ,

(II)

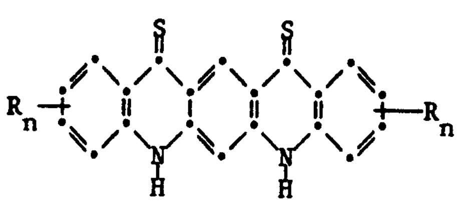 ,

(III)

worin R -F, -Cl, -Br, $C_1$-$C_{18}$-Alkyl oder $C_1$-$C_3$-Alkoxy und n die Zahl 0, 1, 2 oder 3 bedeuten.

**2.** Verbindungen der Formeln I und II gemäss Anspruch 1, worin R und n die im Anspruch 1 angegebene Bedeutung haben.

3. Verbindungen der Formel I gemäss Anspruch 1, worin R -F, -Cl, -Br, $-CH_3$ oder $-OCH_3$ und n die Zahl 0 oder 1 bedeuten.

4. Verfahren zur Herstellung der Verbindungen der Formeln I, II und III gemäss Anspruch 1, dadurch gekennzeichnet, dass man ein entsprechendes Chinacridon, Chinacridonchinon oder Isochinacridon der Formeln I bis III, worin die Schwefelatome durch Sauerstoff ersetzt sind, mit einem Schwefel abgebenden Mittel (Schwefelungsmittel) erwärmt.

5. Verfahren gemäss Anspruch 4, dadurch gekennzeichnet, dass man als Schwefel abgebendes Mittel 2,4-Bis-(4'-methoxyphenyl)-1,3-dithia-2,4-diphosphaetan-2,4-disulfid oder Tetraphosphordekasulfid verwendet.

6. Verfahren gemäss Anspruch 4, dadurch gekennzeichnet, dass man die Umsetzung in einem inerten organischen Lösungsmittel durchführt.

7. Elektrophotographischer Photorezeptor, bestehend aus mindestens einer leitfähigen Unterlage, einer photoleitfähigen Schicht und einer ladungstransportierenden Schicht, dadurch gekennzeichnet, dass in mindestens einer dieser Schichten mindestens eine Verbindung der Formeln I bis III gemäss Anspruch 1 enthalten ist.

8. Elektrophotographischer Photorezeptor gemäss Anspruch 7, dadurch gekennzeichnet, dass die photoleitfähige Schicht mindestens eine Verbindung gemäss Anspruch 1 enthält.

9. Herstellung eines elektrophotographischen Photorezeptors, dadurch gekennzeichnet, dass man eine Verbindung gemäss Anspruch 1 mit einem organischen Bindemittel auf eine leitende Unterlage aufträgt oder im Vakuum aufdampft, die so hergestellte Schicht mit einem organischen Lösungsmittel in flüssiger oder gasförmiger Form behandelt und anschliessend eine zweite Schicht, enthaltend eine aromatische stickstoffhaltige Verbindung, aufbaut.

10. Herstellung eines elektrophotographischen Photorezeptors gemäss Anspruch 9, dadurch gekennzeichnet, dass man ein Lösungsmittel, ausgewählt aus der Gruppe bestehend aus Aceton, Tetrahydrofuran, Dimethylformamid, Methanol, Dimethylsulfoxid, Aethylacetat und Acetonitril, verwendet.

11. In der Masse eingefärbtes hochmolekulares organisches Material enthaltend eine Verbindung der Formel I gemäss Anspruch 1.

## Claims

1. A compound of the formula I, II or III

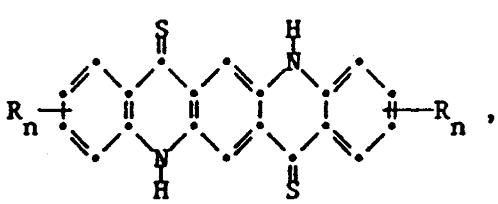 ,

(I)

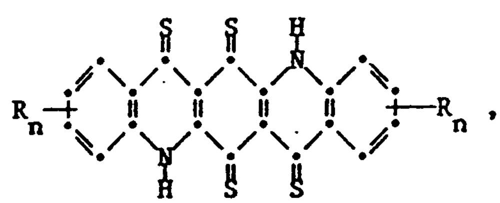 ,

(II)

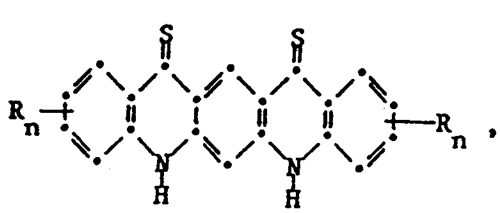 ,

(III)

in which R is -F, -Cl, -Br, $C_1$-$C_{18}$alkyl or $C_1$-$C_3$alkoxy, and n is the number 0, 1, 2 or 3.

**2.** A compound of the formula I or II according to claim 1, in which R and n are as defined in claim 1.

**3.** A compound of the formula I according to claim 1, in which R is -F, - Cl, -Br, $-CH_3$ or $-OCH_3$ and n is the number 0 or 1.

**4.** A process for the preparation of the compounds of the formulae I, II and III according to claim 1, which comprises heating a corresponding quinacridone, quinacridonequinone or isoquinacridone of the formulae I to III, in which the sulfur atoms are replaced by oxygen together with a sulfur-releasing agent (thionating agent).

**5.** A process according to claim 4, wherein the sulfur-releasing agent used is 2,4-bis-(4'-methoxyphenyl)-1,3-dithia-2,4-diphosphetane-2,4-disulfide or tetraphosphorus decasulfide.

**6.** A process according to claim 4, wherein the reaction is carried out in an inert organic solvent.

**7.** An electrophotographic photoreceptor consisting at least of a conductive base, a photoconductive layer and a charge-transporting layer, wherein at least one of these layers contains at least one compound of the formulae I to III according to claim 1.

**8.** An electrophotographic photoreceptor according to claim 7, wherein the photoconductive layer contains at least one compound according to claim 1.

**9.** Production of an electrophotographic photoreceptor, which comprises applying a compound according to claim 1 to a conductive base by means of an organic binder or by vapour deposition in vacuo, treating the layer thus produced with an organic solvent in liquid or gaseous form and subsequently building up a second layer which contains an aromatic nitrogen-containing compound.

**10.** Production of an electrophotographic photoreceptor according to claim 9, wherein the solvent used is selected from the group comprising acetone, tetrahydrofuran, dimethylformamide, methanol, dimethyl sulfoxide, ethyl acetate and acetonitrile.

**11.** A mass-coloured high molecular weight organic material containing a compound of the formula I according to claim 1.

## Revendications

**1.** Composés répondant à l'une des formules I, II et III :

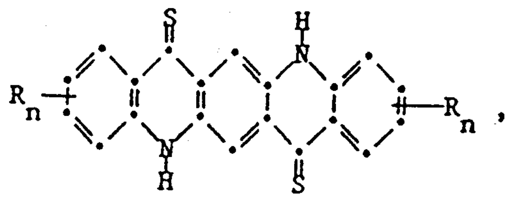

(I)

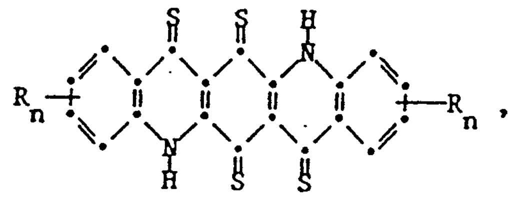

(II)

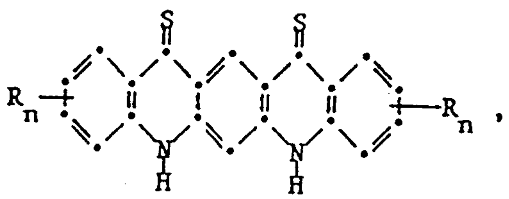

(III)

dans lesquelles R représente -F, -Cl, -Br, un alkyle en $C_1$-$C_{18}$ ou un alcoxy en $C_1$-$C_3$ et n désigne un nombre égal à 0, à 1, à 2 ou à 3.

**2.** Composés de formules I et II selon la revendication 1 dans lesquels R et n ont les significations données à la revendication 1.

**3.** Composés de formule I selon la revendication 1 dans lesquels R représente -F, -Cl, -Br, $-CH_3$ ou $-OCH_3$ et n est égal à 0 ou à 1.

**4.** Procédé pour préparer les composés de formules I, II et III selon la revendication 1, procédé caractérisé en ce qu'on chauffe avec un agent cédant du soufre (agent de sulfuration) une quinacridone, une quinacridone-quinone ou une isoquinacridone correspondante de formules I à III dans lesquelles les atomes de soufre sont remplacés par des atomes d'oxygène.

**5.** Procédé selon la revendication 4 caractérisé en ce qu'on utilise, comme agent cédant du soufre, le disulfure en 2,4 du 2,4-bis-(4-méthoxy-phényl)-1,3-dithia-2,4-diphosphétanneou le décasulfure de tétraphosphore.

**6.** Procédé selon la revendication 4 caractérisé en ce qu'on effectue la réaction dans un solvant organique inerte.

**7.** Photorécepteur électrophotographique constitué d'au moins un support conducteur, d'une couche photoconductrice et d'une couche transporteuse de charges, photorécepteur caractérisé en ce qu'il contient, dans au moins une de ces couches, au moins un composé répondant à l'une des formules I à III selon la revendication 1.

**8.** Photorécepteur électrophotographique selon la revendication 7, caractérisé en ce que la couche photoconductrice contient au moins un composé selon la revendication 1.

**9.** Procédé pour fabriquer un photorécepteur électrophotographique, procédé caractérisé en ce qu'on applique sur un support conducteur un composé selon la revendication 1 avec un liant organique, ou on dépose sur un tel support un tel composé par vaporisation sous vide, on traite la couche ainsi créée par un solvant organique à l'état liquide ou gazeux, puis on créé une seconde couche contenant un composé aromatique azoté.

10. Procédé pour fabriquer un photorécepteur électrophotographique selon la revendication 9, procédé caractérisé en ce qu'on utilise un solvant pris dans l'ensemble constitué par l'acétone, le tétrahydrofuranne, le diméthylformamide, le méthanol, le diméthylsulfoxyde, l'acétate d'éthyle et l'acétonitrile.

11. Matière organique macromoléculaire teinte dans la masse, matière qui contient un composé de formule I selon la revendication 1.